# EUROPÄISCHE PATENTSCHRIFT

(11) **EP 1 636 347 B1**
(45) Veröffentlichungstag und Bekanntmachung des Hinweises auf die Patenterteilung: **30.10.2013**
(21) Anmeldenummer: 04726508.7
(22) Anmeldetag: 08.04.2004
(51) Int. Cl.: C12N 5/074, A61K 35/39

(54) **ISOLIERTE ADULTE PLURIPOTENTE STAMMZELLEN UND VERFAHREN ZU DEREN ISOLIERUNG UND KULTIVIERUNG**
ISOLATED PLURIPOTENT ADULT STEM CELLS AND METHODS FOR ISOLATING AND CULTIVATING THE SAME
CELLULES SOUCHES PLURIPOTENTES ADULTES ISOLEES ET LEURS PROCEDES D'ISOLATION ET DE CULTURE

(30) Priorität: 23.06.2003 DE 10328280
(43) Veröffentlichungstag der Anmeldung: 22.03.2006
(60) Teilanmeldung: 10011672.2
(73) Patentinhaber: Fraunhofer-Gesellschaft zur Förderung der angewandten Forschung e.V., 80686 München (DE)
(72) Erfinder: KRUSE, Charli, 23923 Herrnburg (DE)
(74) Vertreter: Katzameyer, Michael
(86) Internationale Anmeldenummer: PCT/EP2004/003810
(87) Internationale Veröffentlichungsnummer: WO 2005/001072

(56) Entgegenhaltungen:
- WO-A-00/78929
- WO-A1-03/042375
- US-A- 5 462 870
- BONNER-WEIR ET AL: "In vitro cultivation of human islets from expanded ductal tissue" PROCEEDINGS OF THE NATIONAL ACADEMY OF SCIENCES OF USA, NATIONAL ACADEMY OF SCIENCE. WASHINGTON, US, Bd. 97, Nr. 14, 5. Juli 2000 (2000-07-05), Seiten 7999-8004, XP002144480 ISSN: 0027-8424
- GMYR V ET AL: "HUMAN PANCREATIC DUCTAL CELLS: LARGE-SCALE ISOLATION AND EXPANSION" CELL TRANSPLANTATION, ELSEVIER SCIENCE, US, Bd. 10, Nr. 1, 2001, Seiten 109-121, XP001010271 ISSN: 0963-6897

## Beschreibung

Die vorliegende Erfindung betrifft Verfahren zur Isolierung und Kultivierung von adulten pluripotenten Stammzellen.

### Hintergrund der Erfindung

Als Stammzellen werden solche Zellen bezeichnet, welche die Fähigkeit besitzen, sich unbegrenzt zu teilen und sich unter geeigneten Umständen bzw. durch geeignete Stimuli in verschiedene Zellarten differenzieren zu können. Stammzellen besitzen das Potenzial, sich in Zellen mit charakteristischer Gestalt und spezialisierten Funktionen zu entwickeln.

Aufgrund ihrer unterschiedlich ausgeprägten Potenz, sich in verschiedene Zelltypen zu differenzieren, unterscheidet man bisher embryonale von adulten Stammzellen. Es besteht die allgemeine Ansicht, dass mit der Entwicklung von der befruchteten Eizelle über das embryonale Stadium bis zum adulten Organismus die Potenz der Stammzellen abnimmt. Dieser Eigenschaft entsprechend wird die befruchtete Eizelle als totipotent, embryonale Stammzellen als pluripotent und adulte Stammzellen als multipotent bezeichnet.

Totipotente Zellen sind Zellen, aus denen sich ein vollständiger Organismus entwickeln kann; dazu zählen neben der befruchteten Eizelle auch die Zellen der frühen embryonalen Phase. Unter Pluripotenz versteht man, dass die typischerweise aus der inneren Zellmasse von disaggregierten Blastozysten gewonnenen embryonalen Stammzellen in der Lage sind, sich in Zellen aller drei Keimblätter - Mesoderm, Endoderm und Ektoderm - zu differenzieren.

Adulten Stammzellen hingegen wird nach bisheriger Lehrmeinung nur eine Multipotenz zugesprochen, d.h. dass eine Differenzierung in nur geringerem Umfang stattfinden kann. So sollten gewebespezifische Stammzellen nur zur Differenzierung in Zellen desselben Gewebetyps in der Lage sein. In den letzten Jahren musste diese Auffassung zumindestens teilweise korrigiert werden, da Untersuchungen mit adulten Stammzellen, die z.B. aus dem Knochenmark (WO 02/064748) oder aus regenerativem Gewebe (WO 02/057430) erhalten worden waren, belegen, dass unter bestimmten Bedingungen, z.B. Transplantation in ein Zielgewebe, Kultivierung auf einem Feeder Cell Layer und/oder Kultivierung in Gegenwart spezieller Wachstumsfaktoren, auch die Differenzierung einzelner Stammzellen zu Zelltypen verschiedener Keimblätter erreichbar ist. Allerdings kommen auch diese adulten Stammzellen hinsichtlich Selbsterneuerungsvermögen und Differenzierungspotenzial den embryonalen Stammzellen noch nicht gleich. Insbesondere ist die Stabilität dieser Zellinien bei Langzeitkultivierung nicht gewährleistet und die Zellen neigen zu spontaner Differenzierung und unter Umständen auch zur Entartung zu Tumorzellen. Ferner nimmt die Vermehrungsrate mit längerer Kultivierung deutlich ab.

Somit besteht in Anbetracht der ethischen Bedenken bezüglich embryonaler Stammzellen nach wie vor großes Interesse an neuen adulten Stammzellen mit möglichst großem Differenzierungspotenzial und Eigenschaften, die denjenigen der embryonalen Stammzellen gleichkommen.

Der Erfindung liegt daher die Aufgabe zu Grunde, adulte Stammzellen bereitzustellen, die in ihrem Selbsterneuerungsvermögen und Differenzierungspotenzial gegenüber embryonalen Stammzellen keine wesentlichen Unterschiede mehr aufweisen und als tatsächlich pluripotent bezeichnet werden können. Eine damit verbundene Aufgabe ist die Bereitstellung neuer und besonders einfacher Verfahren zur Isolierung und Kultivierung von adulten pluripotenten Stammzellen und davon abgeleiteter differenzierter Zellen..

Diese Aufgaben werden erfindungsgemäß gelöst durch die Bereitstellung isolierter pluripotenter adulter Stammzellen, welche aus exokrinem Drüsengewebe erhalten wurden, durch die erfindungsgemäßen Verfahren zur Isolierung und Kultivierung dieser Stammzellen und davon abgeleiteter differenzierter Zellen.

### Beschreibung der Erfindung

Adulte Stammzellen mit unterschiedlichem Differenzierungspotenzial wurden bereits in zahlreichen Geweben gefunden. Es wurden auch schon mutmaßliche Stammzellen aus dem endokrinen insulin-produzierenden Drüsengewebe des Pankreas isoliert, wohingegen bisher noch nie aus irgendeinem exokrinen Drüsengewebe Zellen isoliert wurden, die als Stammzellen charakterisiert werden konnten.

Es ist daher ausgesprochen überraschend, dass das erfindungsgemäße Verfahren unter erstmaliger Verwendung von exokrinem Drüsengewebe auf besonders einfache Weise die Herstellung adulter Stammzellen ermöglicht, die hinsichtlich ihres Selbstemeuerungsvermögens und ihres Differenzierungspotenzials gegenüber embryonalen Stammzellen keine wesentlichen Unterschiede mehr aufweisen und als pluripotent zu bezeichnen sind.

Das als Ausgangsmaterial verwendete exokrine Drüsengewebe stammt vorzugsweise von einem Wirbeltier, z.B. einem Fisch, Amphibium, Reptil, Vogel oder Säuger, besonders bevorzugt von einem Primaten, insbesondere einem Menschen.

Das erfindungsgemäß verwendete exokrine Drüsengewebe kann von einem erwachsenen Organismus, juvenilen Organismus oder nicht-humanen fötalen Organismus, vorzugsweise einem post-natalen Organismus, stammen. Der Begriff "adult", wie in der vorliegenden Anmeldung verwendet, bezieht sich somit auf das Entwicklungsstadium des Ausgangsgewebes und nicht auf dasjenige des Donororganismus, aus dem das Gewebe stammt. "Adulte" Stammzellen sind nicht-embryonale Stammzellen.

Das exokrine Drüsengewebe wird aus einer Speicheldrüse, Tränendrüse, Talgdrüse oder Schweißdrüse isoliert.. In einer stark bevorzugten Ausführungsform handelt es sich dabei um acinäres Gewebe. Ganz besonders bevorzugt stammt das acinäre Gewebe aus der Ohrspeicheldrüse oder Unterkieferspeicheldrüse.

Die mit dem erfindungsgemäßen Verfahren bereitgestellten Stammzellen sind leicht zu isolieren und in einer stabilen Langzeitkultur ohne Feederzellschicht oder spezielle Zusätze im undifferenzierten Zustand zu halten. Der Begriff Feederzellen, wie in der vorliegenden Anmeldung verwendet, umfasst alle Zellen, die das Wachstum der eigentlich zu kultivierenden Zellen dadurch fördern, dass sie Wachstumsfaktoren freisetzen und/oder eine extrazelluläre Matrix bereitstellen, bzw. die Differenzierung der Stammzellkultur verhindern.

In Stammzellkulturen der Erfinder haben die Zellen nach bisher mehr als 25 Passagen über ein Jahr lang ihre Fähigkeit zur Selbsterneuerung und uneingeschränkten Teilung behalten und die Kulturen sind weiterhin stabil. Nur ein sehr kleiner Teil der Stammzellen (ca. 3 %) differenziert spontan und wird bei einem Mediumwechsel problemlos mit dem alten Medium abgetrennt.

Darüber hinaus können die so produzierten Stammzellen bei der Temperatur des flüssigen Stickstoffs eingefroren gelagert werden und behalten ihre Differenzierbarkeit und Vitalität unverändert bei.

Die erfindungsgemäß erhaltenen adulten Stammzellen können ohne Zusatz spezieller Wachstums- oder Differenzierungsfaktoren auf einfache Weise zur Differenzierung angeregt werden, indem sie unter räumlichen Bedingungen kultiviert werden, welche für einen dreidimensionalen Kontakt der Zellen sorgen. In einer bevorzugten Ausführungsform sind diese Bedingungen die Kultivierung in hängenden Tropfen, wie sie für embryonale Stammzellen bereits beschrieben wurde (Wobus et al., Biomed. Biochim. Acta 47:965-973 (1988). Dieses Verfahren wird nachfolgend in den Beispielen noch näher beschrieben. Es versteht sich jedoch, dass alternative Kultivierungsverfahren, die für den gewünschten dreidimensionalen Kontakt der Zellen sorgen und dem Fachmann bekannt und verfügbar sind, ebenso verwendet werden können. Beispiele für solche alternativen Verfahren sind die Kultivierung in bewegter Suspensionskultur, die Kultivierung in einem elektromagnetischen Feldkäfig oder Laser-Tweezer oder die Kultivierung auf Oberflächen, an denen die Zellen nicht oder schlecht haften. Solche Oberflächen können z.B. Glas, Polystyrol oder mit einer Anti-Adhäsionsschicht behandelte Oberflächen, z.B. PTFE- oder Poly-HEMA-beschichtete Oberflächen, sein.

Unter diesen Bedingungen entwickeln sich spontan dreidimensionale Zellverbände oder Zellaggregate, welche vom Erfinder in Anlehnung an bereits für embryonale Stammzellen beschriebene "embryoid bodies" als "organoid bodies" oder organoide Körper bezeichnet wurden. Diese organoiden Körper oder Organoid Bodies können in Suspensionskulturen oder Adhäsionskulturen überführt und weiter kultiviert werden. Bei ausreichender Nährstoffversorgung wachsen diese organoiden Körper weiter und können Durchmesser von einigen Millimetern oder mehr erreichen. Diese großen Organoid Bodies zeigen eine gewebeartige Struktur und werden in diesem Stadium zur Unterscheidung von den einfachen Zellaggregaten auch als "Gewebekörper" bezeichnet.

Bringt man die Organoid Bodies wieder in Oberflächenkultur, entsteht aus auswachsenden einzelnen Zellen eine zelluläre Monoschicht, aus der Multilayerbereiche hervorgehen, aus denen spontan sekundäre Organoid Bodies mit vergleichbaren Eigenschaften wie denjenigen der primären Organoid Bodies gebildet werden. Die erfindungsgemäßen Organoid Bodies können z.B. bei der Temperatur des flüssigen Stickstoffs eingefroren gelagert werden, ohne ihre Lebensfähigkeit, Vermehrungsfähigkeit, Wachstumsfähigkeit und Differenzierbarkeit zu verlieren.

Zellen, welche aus den organoiden Körpern auswachsen oder isoliert werden, können in die verschiedenen Zelltypen aller drei Keimblätter differenzieren. Zur Differenzierung sind keine Differenzierungsfaktoren notwendig und die Zellen müssen auch nicht transplantiert werden, um zu differenzieren. Es kann jedoch von Vorteil sein, solche Differenzierungsfaktoren einzusetzen, um gezielt größere Mengen eines bestimmten Zelltyps herzustellen. Solche Differenzierungsfaktoren sind im Stand der Technik bekannt und umfassen z.B. bFGF ("basic fibroblast growth factor") zur verstärkten Bildung von Herzzellen und Fibroblasten, VEGF ("vascular endothelial growth factor"), DMSO und Isoproterenol, Fibroblastenwachstumsfaktor 4 (FGF4), Hepatozyten-Wachstumsfaktor (HGF) zur verstärkten Bildung von Herz- und Leberzellen, TGF-beta1 ("transforming growth factor betal") zur verstärkten Bildung von Herzzellen, EGF ("epidermal growth factor") zur verstärkten Bildung von Haut- und Herzzellen, KGF ("keratinocyte growth factor") (machmal zusammen mit Cortison) zur Bildung von Keratinozyten, Retinsäure zur verstärkten Bildung von Nerven-, Herz- und Nierenzellen, beta-NGF ("beta nerve growth factor") zur verstärkten Bildung von Gehirn-, Leber-, Pankreas- und Nierenzellen, BMP-4 ("bone morphogenic protein 4") und Activin-A zur Bildung mesodermaler Zellen generell, sind jedoch nicht darauf beschränkt.

Differenzierte Zellen, die aus den erfindungsgemäß erhaltenen pluripotenten Stammzellen erhältlich sind, umfassen Knochenzellen (Osteoblasten und Osteoclasten), Chondrozyten, Adipozyten, Fibroblasten (z.B. Haut- und Sehnenfibroblasten), Muskelzellen, Endothelzellen, Epithelzellen, hematopoetische Zellen, sensorische Zellen, endokrine und exokrine Drüsenzellen, Gliazellen, neuronale Zellen, Oligodendrozyten, Blutzellen, Darmzellen, Herz-, Lungen-, Leber-, Nieren- oder Pankreaszellen, sind jedoch nicht darauf beschränkt.

Die resultierenden differenzierten Zelltypen können durch histologische, immunozytochemische und elektronenmikroskopische Techniken identifiziert und charakterisiert werden.

Die mit dem erfindungsgemäßen Verfahren erhaltenen adulten pluripotenten Stammzellen bzw. davon abgeleiteten differenzierten Zellen und organoiden Körper haben ein breites Spektrum möglicher Anwendungen auf medizinischen und nicht-medizinischen Gebieten. Beispielsweise können die Zellen einem tierischen oder humanen Patienten verabreicht werden, um eine Verletzung oder einen Krankheitszustand zu behandeln.

Der zu behandelnde Krankheitszustand kann jeder Krankheitszustand sein, von dem zu erwarten steht, dass er mit Stammzellen erfolgreich behandelt werden kann. Solche Krankheitszustände können beispielsweise ein Tumor, eine Lungen-, Leber-, Nierenerkrankung, eine Bindegewebserkrankung, eine kardiovaskuläre Erkrankung, metabolische Erkrankung, neuro-degenerative Erkrankung, Autoimmunerkrankung, Anämie, Hämophilie, Diabetes, Ischämie, Entzündung, eine Infektionskrankheit, ein genetischer Defekt oder Transplantat-Abstoßung sowie Alterungsprozesse sein.

Vorzugsweise sind die verabreichten Zellen autolog, d.h. sie stammen von dem zu behandelnden Patienten, um eine Abstoßungsreaktion zu vermeiden. Die Behandlung kann z.B. darin bestehen, dass die verabreichten Zellen *in vivo* differenzieren, um die Funktion eines geschädigten oder fehlenden Organs oder Gewebes im Körper eines Patienten zu übernehmen. Das Organ oder Gewebe kann beispielsweise aus Knochenmark, Blut, Milz, Lunge, Niere, Blase, Darm, Gehirn, Fettgewebe, Bindegewebe, Muskelgewebe, Herz, Blutgefäßen, Pankreas, ZNS, peripherem Nervensystem, Haut, insbesondere Schleimhäuten, ausgewählt sein.

In einer bevorzugten Ausführungsform stärkt die Behandlung das Immunsystem des Patienten oder stellt es wieder her. In einer weiteren Ausführungsform besteht die Behandlung darin, dass Blut oder eine andere Körperflüssigkeit eines Patienten außerhalb des Körpers mit den Stammzellen oder davon abgeleiteten Zellen, z.B. Leber- oder Nierenzellen, in Kontakt gebracht wird, um z.B. die Körperflüssigkeit von Giftstoffen zu befreien oder auf andere Weise einer therapeutischen oder diagnostischen Behandlung zu unterziehen, und dann die Körperflüssigkeit wieder in den Körper rückgeführt wird.

Die verabreichten erfindungsgemäß erhaltenen Stammzellen in einem Behandlungsverfahren können auch als gefahrloses Vehikel für ein therapeutisches Agens dienen. Das therapeutische Agens kann z.B. DNA, RNA, ein Protein oder Peptid oder ein niedermolekularer Arzneiwirkstoff sein.

Insbesondere können die Stammzellen gentechnisch manipuliert werden, um bestimmte Eigenschaften aufzuweisen, und dann zur Gentherapie eingesetzt werden. Solche Eigenschaften können z.B. verringerte Antigenizität, Produktion gewünschter DNA, RNA, Proteine, fehlende Produktion unerwünschter DNA, RNA, Proteine, etc. sein.

Die Verabreichung der Zellen kann nach jedem im Stand der Technik bekannten und für diesen Zweck geeigneten Verfahren erfolgen. Geeignete Verfahren schließen ein, sind jedoch nicht beschränkt auf, lokale Injektion, systemische Injektion, parenterale Verabreichung, orale Verabreichung oder intrauterine Injektion in einen Embryo.

In der Regel werden die zur Behandlung eingesetzten Stammzellen oder davon abgeleiteten differenzierten Zellen in einer pharmazeutischen Zusammensetzung vorliegen. Eine solche Zusammensetzung kann neben den Zellen eine physiologisch annehmbare Matrix oder einen physiologisch annehmbaren Träger enthalten. Die Art der Matrix bzw. des Trägers wird unter anderem von dem vorgesehenen Verabreichungsweg abhängen. Geeignete Matrices bzw. Träger sind im Stand der Technik bekannt. Ferner kann die pharmazeutische Zusammensetzung gegebenenfalls weitere geeignete Hilfsstoffe oder Wirkstoffe enthalten. Eine weitere mögliche Anwendung ist die Verwendung der Stammzellen, Stammzellkulturen oder der organoiden Körper oder der daraus erhaltenen differenzierten Zellen zur Entwicklung gewebeähnlicher oder organähnlicher multizellulärer Systeme *in vitro.* In einer bevorzugten Ausführungsform umfassen die multizellulären Systeme dabei verschiedene Zellarten. Diese multizellulären Systeme können dann beispielsweise transplantiert werden oder extrakorporal, z.B. zur Blutwäsche oder zur Produktion gewünschter Substanzen, eingesetzt werden.

Eine noch weitere mögliche Anwendung ist die Verwendung der Stammzellen, Stammzellkulturen oder der organoiden Körper oder der daraus erhaltenen differenzierten Zellen oder multizellulären Systeme als *in vitro*-System zum Testen von Chemikalien, insbesondere zum Screenen von Arzneiwirkstoffen oder Kosmetika und/oder zur Analyse der Wirkung bekannter oder potenzieller Wirkstoffe oder Giftstoffe oder Mutagene auf die jeweiligen Zellen. Diese Wirkung kann beispielsweise eine Veränderung des Proliferationsvermögens, Differenzierungsvermögens oder der Lebensfähigkeit der Zellen sein.

Ebenfalls eine mögliche Anwendung ist die Bereitstellung komplexer zellulärer Systeme zur Analyse zellulärer Interaktionen.

Eine noch weitere Anwendung ist die Verwendung der aus den Stammzellen erhaltenen differenzierten Zellen zur Produktion gewünschter Substanzen *in vitro.*

Eine spezielle Anwendung für nicht-humane adulte Stammzellen ist die Verwendung der adulten Stammzellen oder davon erhaltenen differenzierten Zellen für das therapeutische oder reproduktive Klonieren. Dabei werden beispielsweise die adulten Stammzellen oder davon abgeleiteten differenzierten Zellen oder daraus isolierte Zellkerne in entkernte nicht-humane Oozyten eingebracht und die Oozyten unter Bedingungen kultiviert, welche zur Entwicklung von Blastozyten führen. Aus den Blastozyten können dann entweder embryonale Stammzellen gewonnen werden, welche in bekannter Weise zur Differenzierung in einen gewünschten Zelltyp veranlasst werden können (therapeutisches Klonieren), oder die Blastozyten können bis zur Entwicklung eines Embryos gebracht werden, der einem weiblichen Tier implantiert und nach der entsprechenden Gestationszeit natürlich geboren werden kann (reproduktives Klonen).

In einer Ausführungsform werden die Stammzellen oder davon abgeleiteten Organoid Bodies oder differenzierten Zellen in Form eines Kits bereitgestellt. Ein solcher Kit kann die Zellen als solche oder als pharmazeutische Zusammensetzung wie oben definiert umfassen. Ferner kann der Kit weitere Hilfsmittel, z.B. zur Verabreichung der Zellen, oder Reagenzien, z.B. Reagenzien zur Kultivierung der Zellen oder diagnostische Reagenzien, umfassen.

### FIGURENBESCHREIBUNG

- Fig. 1: zeigt schematisch die Kultivierung der Stammzellen in Oberflächenkultur und in hängenden Tropfen sowie die Bildung und weitere Kultivierung von organoiden Körpern.
- Fig. 2: zeigt den immunhistologischen Nachweis von Epithel- und Drüsenzellen mit entodermalen Eigenschaften, die aus Stammzellen aus Ratten-Pankreas hergestellt wurden. Die Zellen wurden mit Antikörpern gegen Cytokeratin und Amylase angefärbt. Zusätzlich wurden die Zellkerne mit DAPI angefärbt.
- Fig. 3: zeigt den immunhistologischen Nachweis von differenzierten Muskelzellen mit mesodermalen und Nervenzellen mit ektodermalen Eigenschaften, die aus Stammzellen aus Ratten-Pankreas hergestellt wurden. Die Zellen wurden mit Antikörpern gegen α-smooth-muscle-actin (SMA) und Neurofilament (NF) angefärbt. Zusätzlich wurden die Zellkerne mit DAPI angefärbt.

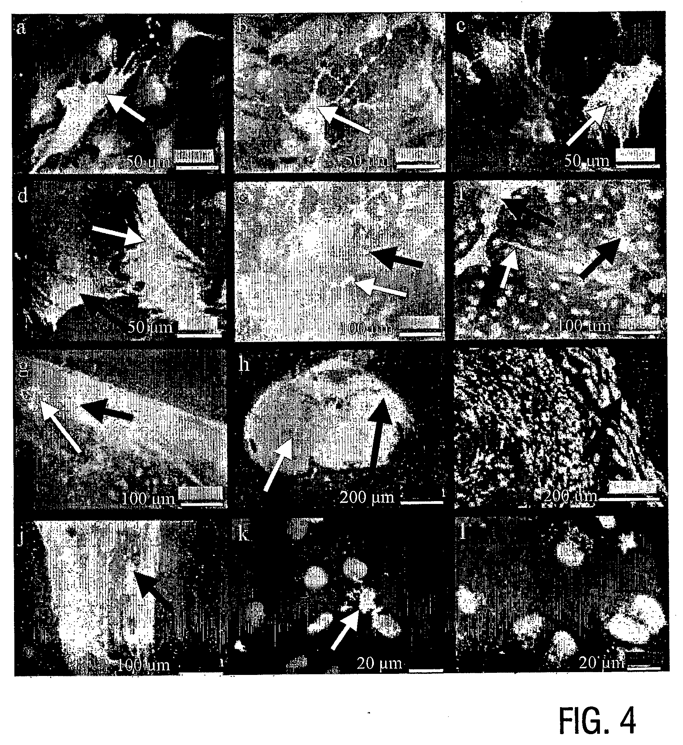

- Fig. 4: zeigt die Expression von Markern neuronaler, glialer und glatter Muskelzellen sowie von Amylase und Insulin. a,b: PGP 9.5-markierte Nervenzellen zeigen multipolare Ausläufer, welche zahlreiche Varikositäten zeigen. c,d: das Neurofilament-Ssystem (helle Pfeile, im Originalfoto grün markiert) reicht durch das Pericaryon in die cytoplasmatischen Ausläufer. In enger Nachbarschaft liegen GFAP-immunreaktive Gliazellen (dunkle Pfeile, im Originalfoto rot markiert). e,f: α-SMA-markierte Zellen (dunkle Pfeile, im Original rot) und NF-markierte Nervenzellen (helle Pfeile, im Original Grün) bilden ein primitives neuro-muskuläres Netzwerk (e), wobei Kontakte über beträchtlich lange Entfernungen etabliert werden (f). g: Immunfärbung von GFAP (dunkle Pfeile, im Original rot) und NF (helle Pfeile, im Original grün) in 3 Wochen alten OB mit Konzentrationen an Nerven- und Gliazellen. h: Immunfärbung von α-SMA (dunkle Pfeile, im Original rot) und NF (helle Pfeile, im Original grün) in 3 Wochen alten OBs in einem fortgeschrittenen Stadium der Bildung eines neuromuskulären Netzwerks. i,j: in Querschnitten von 8 Wochen alten OBs wurden für NF immunreaktive Zellen in direkter Nachbarschaft von Zellen gefunden, die immunreaktiv für α-SMA waren, ähnlich wie in nativen Geweben. k: eine Untergruppe von Zellen zeigt eine positive Färbung für Amylase (helle Pfeile, im Original grün). Die Amylase-enthaltenden Zellen sind kreisförmig gruppiert, wie anhand der topographischen Anordnung ihrer Kerne festgestellt, und speichern das exokrine Sekret innerhalb der apikalen Zellpole und zeigen damit morphologische Merkmale von exokrinen pankreatischen Acini. 1: Eine weitere Zelluntergruppe enthält granuläre Vesikel mit Immunreaktivität für Insulin. Das endokrine Produkt ist nicht homogen verteilt, sondern in einem polaren Muster gespeichert. Die Kerne sind mit DAPI kontrastgefärbt (im Original blau).

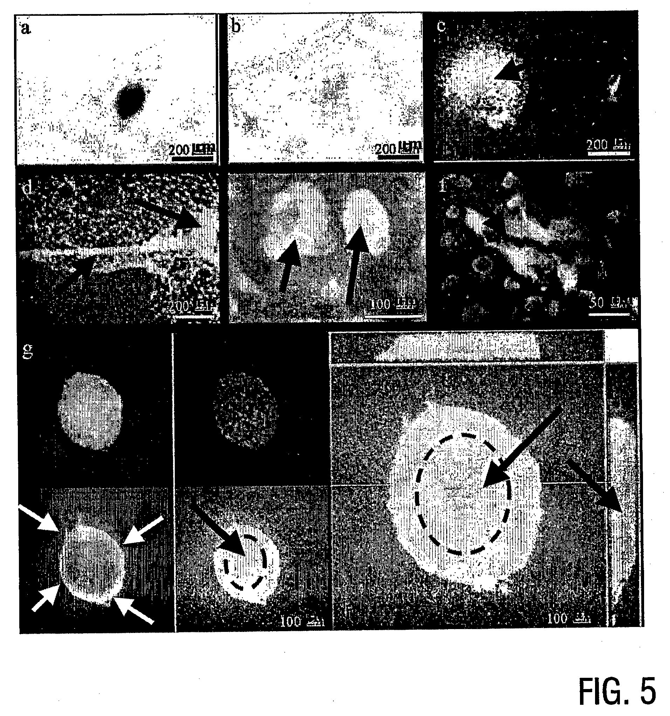

- Fig. 5: zeigt die Expression von extrazellulären Matrixkomponenten und Cytokeratinen. a,b: Globuläre (a) und fibrilläre (b) Speicher von Proteoglycanen ergaben eine Färbung mit Alcianblau. c-e: Die globulären (c) und fibrillären (d) Bereiche sind immunreaktiv für das Knorpelmatrixprotein Kollagen II. Dicht verteilte Zellen werden neben der sich entwickelnden extrazellulären Matrix beobachtet. Zwei individuelle Zellen (e) zeigen eine cytoplasmatische Markierung von Kollagen II. f: Zellen, die für Cytokeratine immunreaktiv sind, sind in Clustern angeordnet. g: konfokale Laserscanningmikroskopie eines OB. Die Kollagen II-Immunreaktivität (dunkle Pfeile, im Original rot markiert) nimmt zur Mitte des OB hin zu. Vigilin-immunreaktive Zellen (helle Pfeile, im Original grün markiert) sind hauptsächlich am äußeren Rand des OB lokalisiert, was deren hohe Translationsaktivität anzeigt. Die Kerne sind mit DAPI kontrastgefärbt.

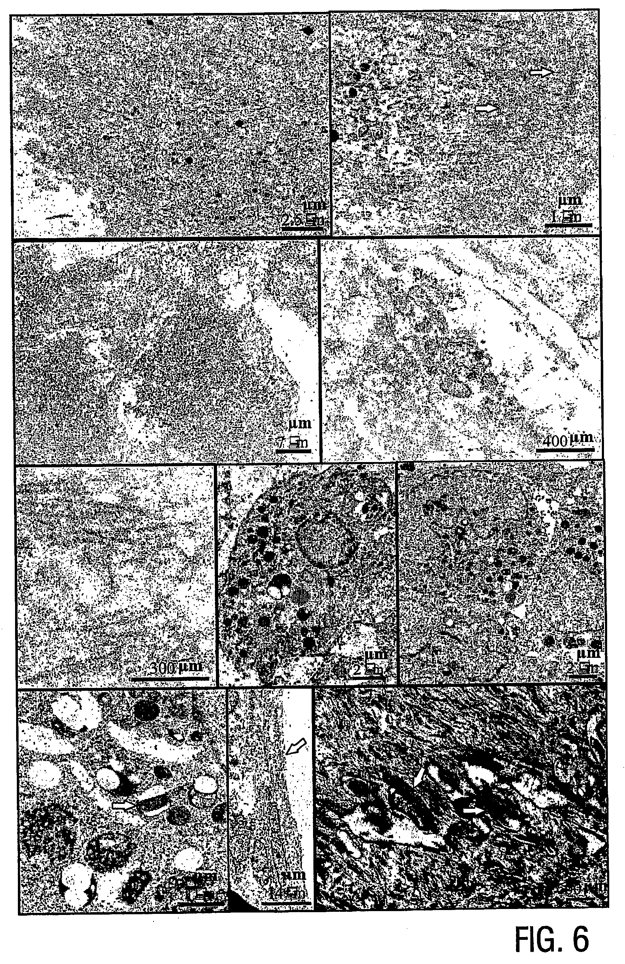

- Fig. 6: zeigt die Transmissionselektronenmikroskopie differenzierter OB. a-c: glatte Muskelzellen mit Myofilamenten. Das Myofilamentsystem erstreckt sich über das Cytoplasma in disseminiertenBündeln (a) und zeigt typische dichte Körper (Pfeile) (b). Die Myoblasten zeigen sternförmige Zellausläufer, die ein verbindendes Netzwerk bilden (c). d: Zellausläufer mit einer Ansammlung von zahlreichen Vesikeln kleiner Größe, die höchstwahrscheinlich Nervenfaservarikositäten entsprechen. e: Kollagen- und Retikulumfasern. Das typische gestreifte Muster (periodische Banden) von Kollagenfasern ist in längsgeschnittenen Fibrillen wahrnehmbar. f-h: Sekretorische Zellen zeigen elektrodichte Vesikel. Die Vesikel nehmen nicht das ganze Cytoplasma ein, sondern sind an einem Zellpol gespeichert (f). Häufig kontaktieren sekretorische Zellen einander, um acinusartige Strukturen zu bilden (g). Ein Untergruppe von sekretorischen Zellen enthält Vesikel (Pfeil) die Ultrastruktur-Merkmalen von endokrinen Granula entsprechen (h), z.B. Beta-Granula von insulinproduzierenden Zellen. 1: Beginn der Ausbildung einer Epitheloberfläche (Pfeil) in acht Wochen alten OBs. j: typische Zellkontakte zwischen Keratinozyten und Desmosomen (Pfeile)

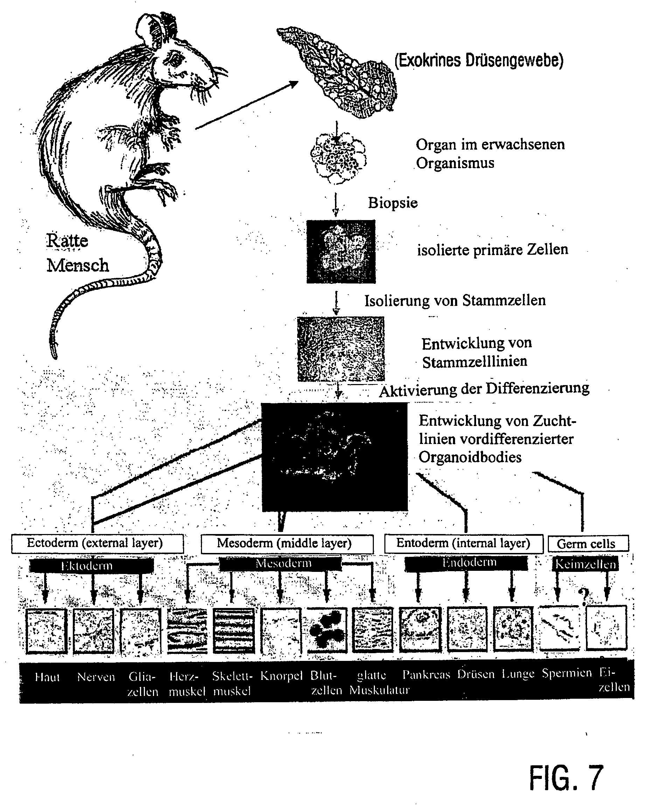

- Fig. 7: zeigt schematisch die Hauptstufen der erfindungsgemäßen Verfahren zur Isolierung und Kultivierung von adulten Stammzellen und zur Gewinnung organoider Körper und eines breiten Spektrums differenzierter Zellen daraus.

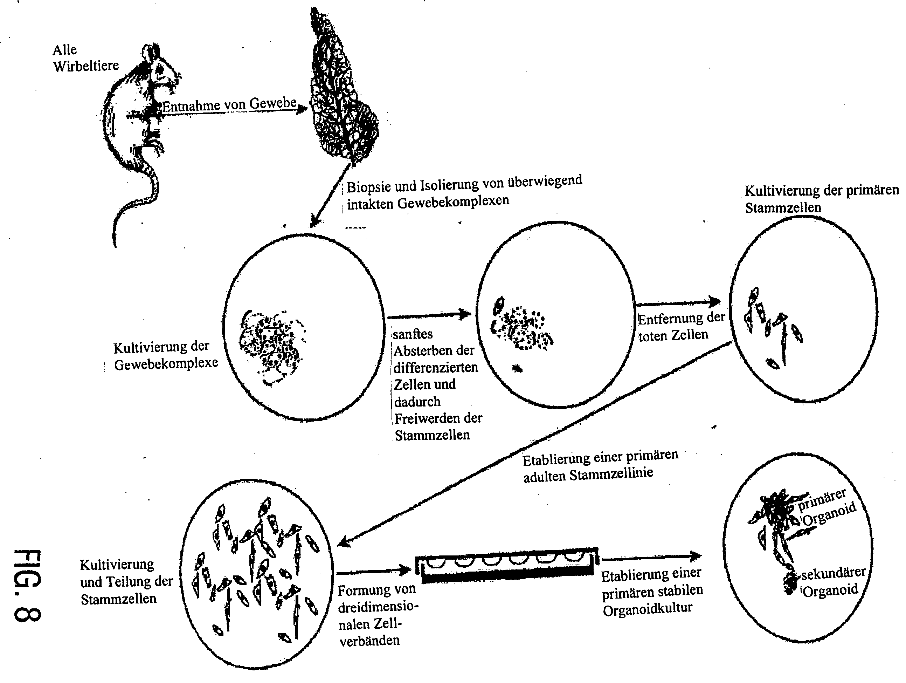

- Fig. 8: zeigt schematisch verschiedene Einzelschritte der erfindungsgemäßen Verfahren zur Isolierung und Kultivierung von adulten Stammzellen und zur Gewinnung organoider Körper.

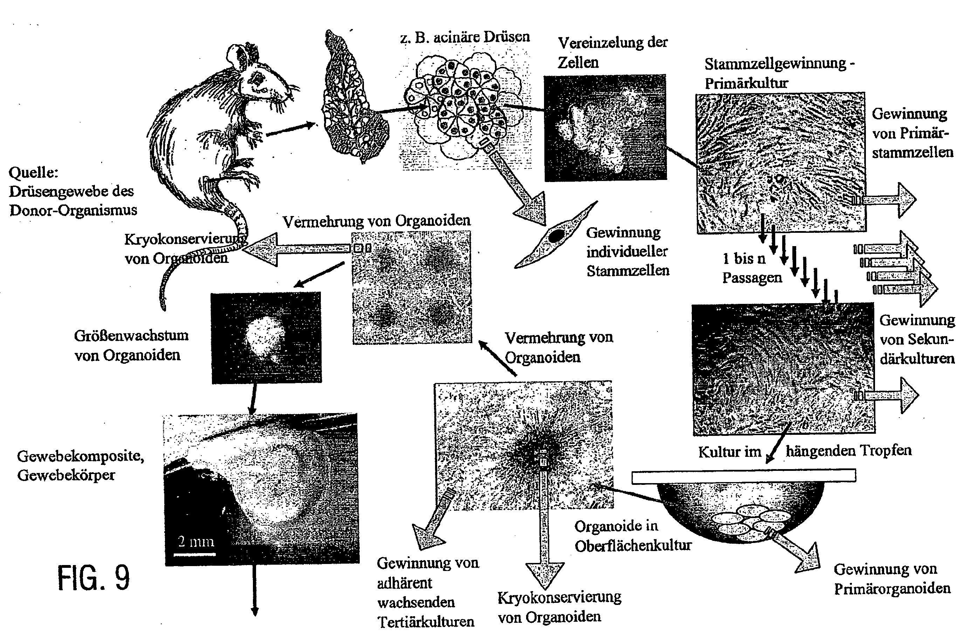

- Fig. 9: zeigt mikroskopische Aufnahmen der Stammzellen und organoiden Körper nach verschiedenen Einzelschritten der erfindungsgemäßen Verfahren zur Isolierung und Kultivierung von adulten Stammzellen und zur Gewinnung organoider Körper.

In einer besonders bevorzugten Ausführungsform der vorliegenden Erfindung werden die adulten Stammzellen aus acinärem Gewebe erhalten.

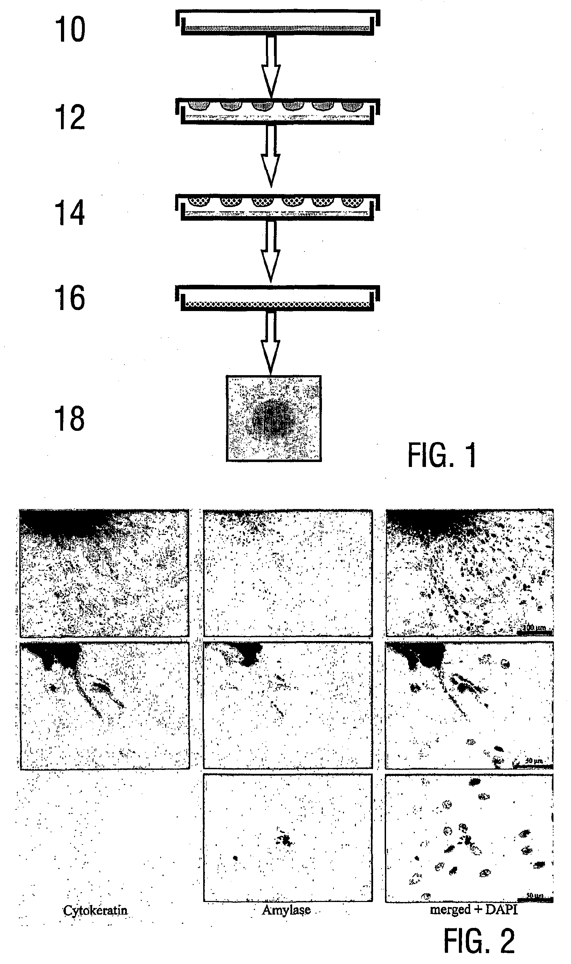

Entsprechend dem in Fig. 1 dargestellten Schema wird zur Gewinnung der Zellen acinäres Gewebe - bevorzugt der Ohrspeicheldrüse - mechanisch und enzymatisch zerkleinert in Kultur genommen [Schritt 10 in Fig. 1]. Entgegen den Angaben von Bachem et al., Gastroenterol. 115:421-432 (1998), und Grosfils et al., Res. Comm. Chem. Pathol. Pharmacol. 79:99-115 (1993), werden keine Gewebeblöcke kultiviert, aus denen Zellen auswachsen sollen, sondern unter der Bedingung, dass die Zellverbände der Acini weitestgehend intakt bleiben, das Gewebe stärker zerkleinert.

Über mehrere Wochen werden diese Zellen und Zellverbände in Kulturgefäßen kultiviert. Alle 2-3 Tage wird das Medium gewechselt, wobei alle differenzierten Zellen entfernt werden. Bei den in Kultur persistierenden Zellen handelt es sich um undifferenzierte Zellen mit uneingeschränkter Teilungsfähigkeit.

Ähnliche Zellen sind unter gleichen Bedingungen aus dem Pankreas isoliert und beschrieben und als eine Art Myofibroblasten bzw. pankreatische Sternzellen bezeichnet worden (Bachem et al., 1998). Im Gegensatz zu den Zellen der vorliegenden Erfindung konnte eine uneingeschränkte Teilungsfähigkeit jedoch nicht beobachtet werden. Weiterhin konnten diese Zellen auch nicht unbegrenzt passagiert werden, ohne an Vitalität zu verlieren.

In einem zweiten Schritt [12] werden ungefähr 400 bis 800 Zellen in je 20 µl Medium in hängenden Tropfen kultiviert. Dazu werden die Tropfen auf Deckel von bakteriologischen Petrischalen gegeben, umgedreht und über die mit Medium gefüllte Petrischale gelegt, sodass die Tropfen nach unten hängen.

Durch diese Art der Kultivierung bilden sich innerhalb von 48 h als "organoid bodies" bezeichnete Zellaggregate [14], die für ungefähr 6 Tage in eine Suspensionskultur umgesetzt werden [16]. Die Teilansicht [18] aus Fig. 1 zeigt eine mikroskopische Aufnahme eines solchen Organoid Body.

Die in Suspensionskultur wachsenden Organoid Bodies bilden neue Organoid Bodies, die auch bei Einzelzellen die Bildung von neuen Organoid Bodies induzieren. Die Zellen sind sowohl als Organoid Bodies als auch als Einzelzellen einfrierbar und behalten dabei ihre Vitalität und ihr Differenzierungspotenzial.

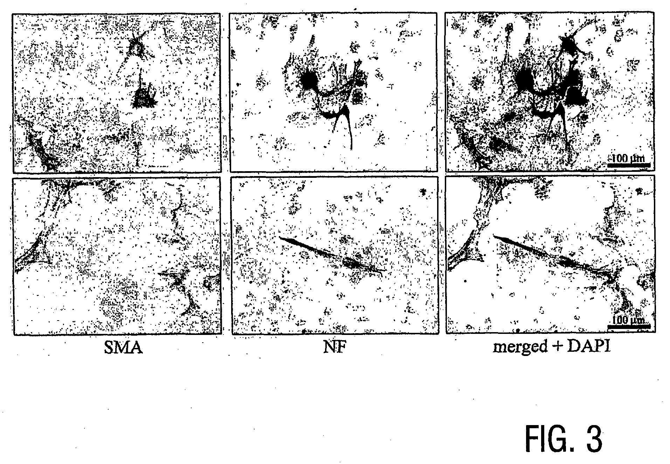

Die Figuren 2-6 zeigen mikroskopische und elektronenmikroskopische Aufnahmen von differenzierten Zellen, die aus solchen Organoid Bodies erhalten wurden.

Dabei konnte z.B. die Bildung eines neuro-muskulären Netzwerks beobachtet werden: Aus OBs erhaltene Zellen exprimierten stark α-SMA ("smooth-muscle-actin") (Fig. 4e-f). Die Anwesenheit verbreiteter Bündel von Myofilamenten, welche sich über das Cytoplasma erstreckten, wurde durch Elektronenmikroskopie bestätigt (Fig. 6a-c). Ferner wurden Zellen identifiziert, welche für den pan-Neuronenmarker PGP 9.5 und für Neurofilamente (NF) immunreaktiv waren. Das Neurofilamentsystem reichte vom Pericaryon in die radialen cytoplasmatischen Ausläufer (Fig. 4c,d). PGP 9.5-immunreaktive Zellen zeigten zahlreiche Varikositäten entlang ihrer verzweigten Ausläufer (Fig. 4a,b, 6d) und entsprachen damit typischen morphologischen Merkmalen autonomer Nervenfasern. Zellen, die für GFAP ("glial fibrillary acidic protein") immunreaktiv waren, befanden sich in enger Nachbarschaft zu Zellen, die neuronale Marker exprimierten (Fig. 4c,d). Häufig erstreckten sich die flamentösen Proteine nicht durch das gesamte Cytoplasma, sondern waren auf Gebiete neben den Nervenzellen beschränkt. Ferner waren glatte Muskelzellen und Nervenzellen nicht willkürlich verstreut, sonden bildeten zusammenhängende Netzwerke mit unschwer unterscheidbaren Verbindungen (Fig. 4e,f). Nervenfaserausläufer erstreckten sich über beträchtlich große Entfernungen, um benachbarte glatte Muskelzellen als ihre mutmaßlichen Ziele zu kontaktieren. Somit zeigten die beiden Zelltypen aufgrund ihrer topographischen Anordnung Merkmale eines primitiven neuromuskulären Netzwerks. Bei 3 Wochen alten OBs wurde eine beginnende Bildung von gewebeähnlichen Strukturen festgestellt (Fig. 4g-j). Hier wurde ein Cluster von faserförmigen Nervenzellen in Kontakt mit Gliazellen gefunden (Fig. 4g) oder weiter entwickelt zu einem dreidimensionalen neuro-muskulären Netzwerk (Fig. 4h), was in Querschnitten von 8 Wochen alten OBs bestätigt wurde (Fig. 4i,j).

Nachweis der Expression exokriner und endokriner pankreatischer Proteine: Immunhistochemische Anfärbungen zeigten, dass zelluläre Untergruppen positiv für Amylase waren (Fig. 4k). Das Immunreaktionssignal war auf klar unterscheidbare Vesikel inerhalb des apikalen Cytoplasmas beschränkt. Darüber hinaus waren die meisten Zellcluster, die für Amylase immunreaktiv waren, in Kreisen angeordnet, wobei die sekretorischen Vesikel eine Position zur Mitte hin aufwiesen, welches eine morphologische Anordnung ähnlich der von exokrinen Pankreas-Acini ist. Andere zelluläre Untergruppen zeigten Immunreaktivität für Insulin (Fig. 4l). Ähnlich wie bei den Amylase-positiven Zellclustern, war das sekretorische Produkt in vesikulären Strukturen gespeichert, die an einem Zellpol konzentriert waren. Die Anwesenheit sekretorischer Zellen wurde durch Elektronenmikroskopie bestätigt, welche dicht verteilte elektrodichte Partikel zeigte, wie sie charakteristisch für exkretorische oder inkretorische Funktionen sind (Fig. 6f-h). Ebenfalls beobachtet wurde eine Differenzierung in chondrogene Zellen und Epithelzellen: Nach einer Wachstumsperiode von zwei Monaten zeigten OBs chondrogene Eigenschaften. Eine Alcianblaufärbung offenbarte Bereiche mit hohen Konzentrationen an Proteoglycanen (Chondroitinsulfat), die entweder als globuläre (Fig. 5a) oder fibrilläre (Fig. 5b) Ablagerungen vorkamen. Immunhistochemische Anfärbungen mit Antikörpern, die gegen das Knorpelmatrixprotein Kollagen II gerichtet waren, belegten zusätzlich die chondrogene Aktivität innerhalb dieser globulären (Fig. 5c) und fibrillären (Fig. 5d) Bereiche. Die Immunreaktivität war am höchsten in der Mitte der zellulären Aggregate, die am wahrscheinlichsten Bereichen von sich entwickelnder extrazellulärer Knorpelmatrix entsprach. Diese Beobachtung wurde durch konfokale Mikroskopie bestätigt (Fig. 5g): während die Menge der Kollagen-Speicher zur Mitte der zellulären Agregate hin zunahm, waren die Randbereiche durch aktiv translatierende Zellen charakterisiert, wie durch deren hohe Vigilin-Expression demonstriert, die gewöhnlich bei Zellen mit aktiver Translationsmaschinerie, z.B. Kollagen-synthetisierenden Chondrozyten oder in Fibroblasten während der Chondroinduktion, gefunden wird. Typische einzelne Kollagen II-translatierende Chondrozyten wurden auch in auswachsenden Zellen von OBs beobachtet, die eine Kollagen II-haltige Matrix produzierten, welche die Einzelzellen umgab (Fig. 5e). Eine Ultrastruktur-Untersuchung dieser Bereiche konnte klar ein Netzwerk von Retikulumfasern und Kollagenfasern zeigen, wobei die letzteren durch ihre charakteristisches Bandenmuster identifiziert wurden (Fig. 6e). Einige Zellen exprimierten neben mesenchymalen Markern auch mehrere Cytokeratine, was deren Potenzial zur Differenzierung in Epithelzellen anzeigt. Jedoch wurden Zellen, die für Cytokeratine immunreaktiv waren, weniger häufig gefunden als Zellen, die Marker von glatten Muskelzellen und Neuronen exprimierten. Typischerweise waren sie in Clustern angeordnet, die innerhalb der OBs disseminiert waren (Fig. 5f). Durch elektronenmikroskopische Untersuchungen wurden typische Zellkontakte zwischen Keratinozyten gefunden (Fig. 6j) und bei 8 Wochen alten OBs wurden Epithelzellen auf der Oberfläche gefunden, welche aus dem Zellkulturmedium in die Luft wuchs.

Insgesamt konnten bisher z.B. folgende Marker für spezifische Zellen positiv getestet werden: PGP 9.5 und NF für Nervenzellen, S 100 und GFAP für Gliazellen, SMA für Muskelzellen (bzw. Myofibroblasten), Kollagen Typ II für Knorpelzellen, Amylase und Trypsin für exokrine Drüsenzellen, Insulin für endokrine Drüsenzellen, Vigilin für stark translatierende Zellen und Cytokeratin für epidermale Zellen. Neben den lichtmikroskopischen Untersuchungen konnten auch elektronenmikroskopisch verschiedene Zelltypen morphologisch charakterisiert werden, sowie Zell-Zell-Kontakte als Zeichen für zelluläre Interaktionen gefunden werden.

Es wurden bisher u.a. glatte Muskelzellen, Neuronen, Gliazellen, Epithelzellen, Fettzellen, Herzzellen, Nierenzellen, Fibroblasten (z.B. Haut- und Sehnenfibroblasten), Chondrozyten, endokrine und exokrine Drüsenzellen und damit Zelltypen aller drei Keimblätter nachgewiesen.

In den folgenden, nicht-beschränkenden Beispielen soll die vorliegende Erfindung näher erläutert werden.

Die allgemeinen Arbeitsanweisungen, wie sie für Verfahren zur Kultivierung von tierischen Zellen und insbesondere von Säugetierzellen gebräuchlich sind, sind zu beachten. Eine sterile Umgebung, in der das Verfahren durchgeführt werden soll, ist - auch wenn hierzu keine weitere Beschreibung erfolgt - in jedem Fall einzuhalten. Folgende Puffer und Medien wurden verwendet:

| | | |
|---|---|---|
| HEPES-Stammlösuns (pH 7.6) | 2,3 83 g HEPES auf 100 ml A. *bidest*, | |
| HEPES-Eagle-Medium (pH 7,4) | 90 ml Modified Eagle Medium (MEM) | |
| | 10 ml HEPES-Stammlösung | |
| | | |
| Isolationsmedium (pH 7,4) | 32 ml HEPES-Eagle-Medium | |
| | 8 ml 5% BSA in A. bidest. | |
| | 300 µl 0,1 M CaCl2 | |
| | 100 µl Trasylol (200.000 KIE) | |
| Digestionsmedium (pH 7,4) | 20 ml Isolationsmedium | |
| | 4 ml Kollagenase (Kollagenase NB 8 von Serva) | |
| | | |
| Inkubationsmedium | Dulbecco's Modified Eagle Medium (DMEM) | |
| Nährmedium | Dulbecco's Modified Eagle Medium (DMEM) | |
| | DMEM | + 4500 mg/l Glucose |
| | | + L-Glutamin |
| | | - Pyruvat |
| | + 20 % FKS (inaktiviert) + 1 ml/100 ml Pen/Strep | |
| | (10000 E/10000 µg/ml) | |
| | oder | |
| | DMEM + 10 % Eigenplasma + 1 ml/100 ml Pen/Strep, | |
| | vor Gebrauch auf 37°C erwärmen | |
| Differenzierungsmedium | 380 ml DMEM | |
| | 95 ml 30 min bei 54 °C inaktiviertes FKS | |
| | 5 ml Glutamin (GIBCO BRL) | |
| | 5 ml (3,5 µl ß-Mercaptoethanol auf 5 ml PBS) | |
| | 5 ml Non-essential amino acids (GIBCO BRL) | |
| | 5 ml Penicillin/Streptomycin (GIBCO BRL) | |
| | (10000 E/10000 µg/ml) | |

Statt fötalem Kalbserum (FKS) im Nährmedium und Differenzierungsmedium kann gegebenenfalls auch Eigenplasma oder, weniger bevorzugt, Eigenserum des Gewebedonors verwendet werden. Dies ist insbesondere dann von Bedeutung, wenn der Gewebedonor mit dem späteren Empfänger der Stammzellen oder davon abgeleiteten differenzierten Zellen identisch ist. Eine solche autologe Behandlung ist zur Verhinderung einer eventuellen Abstoßungsreaktion bevorzugt.

Das Nährmedium kann als Basismedium statt des verwendeten DMEM-Mediums auch ein anderes für die Kultivierung von eukaryotischen Zellen, insbesondere Säugerzellen, bekanntes, geeignetes Basismedium enthalten, in dem die differenzierten Zellen absterben und sich die gewünschten Stammzellen vermehren. Auch Isolationsmedium, Inkubationsmedium und Differenzierungsmedium können ein anderes übliches und geeignetes Basismedium enthalten.

Die folgenden Referenzbeispiele 1 und 2 beschreiben detailliert zwei Arbeitsprotokolle zur Isolierung und Kultivierung adulter pluripotenter Stammzellen aus acinärem Gewebe des Pankreas (nicht Teil der Erfindung). Beispiel 1 beschreibt ein entsprechendes analoges Protokoll für die erfindungsgemäße Isolierung aus acinärem und tubulösem Gewebe der Speicheldrüse.

### REFERENZBEISPIEL 1

### 1. Präparation des Gewebes und Isolierung der Zellen

In den *Ductus pancreaticus* von 2-3 Jahre alten Ratten werden mittels einer Spritze und einer stumpfen Kanüle bei der Ratte 10 ml Digestionsmedium langsam und luftblasenfrei injiziert. Das gesamte Pankreas wird dadurch aufgeblasen und kann so besser herauspräpariert werden. Das Pankreas wird dann in ein Becherglas überführt und weitere 5 ml Digestionsmedium dazugegeben. Nachdem man das Fettgewebe und Lymphknoten entfernt hat, wird das Gewebe im Becherglas mit einer feinen Schere sehr fein zerkleinert, oben schwimmendes Fettgewebe abgesaugt und die Suspension wird abschließend 1 min mit Carbogen begast (wenn nötig wiederholen) und für 20 min bei 37 °C mit Alufolie bedeckt in einem Schüttler bei 200 Zyklen/min inkubiert. Danach saugt man das Medium vorsichtig ab, zerkleinert erneut mit einer Schere das Gewebe und wäscht die Gewebestücke zweimal mit je 10 ml Isolationsmedium und gibt wieder 5 ml Digestionsmedium zum Gewebe hinzu.

Nach erneutem Begasen mit Carbogen für etwa 1 min und Inkubation für 15 min bei 37 °C in einem Schüttler bei 200 Zyklen/min werden die Gewebestücke durch sukzessives Aufziehen in jeweils einer 10 ml, 5ml, 2 ml und 1 ml Glaspipette zerkleinert und durch einlagiges Filtergewebe gepresst. Die so vereinzelten Zellen werden nun fünfmal in Inkubationsmedium gewaschen (37 °C), mit Carbogen begast und jedes Mal 5 min bei 90 g zentrifugiert. Das zuletzt erhaltene Pellet wird in Inkubationsmedium resuspendiert, begast und auf Gewebekulturschalen verteilt.

### 2. Kultivierung der Zellen

Die Gewebekulturschalen mit den isolierten Zellen werden im Brutschrank bei 37°C und 5 % CO₂ kultiviert. Alle 2-3 Tage wird das Medium gewechselt. Dabei werden alle differenzierten Zellen entfernt.

Am siebenten Tag in Kultur werden die Zellen mit einer Lösung bestehend aus 2 ml PBS, 1 ml Trypsin und 2 ml Inkubationsmedium passagiert. Dabei lösen sich die Zellen vom Boden der Kulturschale. Die Zellsuspension wird 5 Minuten zentrifugiert, der Überstand abgesaugt und die Zellen in 2 ml Inkubationsmedium resuspendiert, auf eine mittlere Zellkulturflasche überführt und 10 ml Inkubationsmedium dazugegeben. Der Medienwechsel erfolgt alle drei Tage.

Am vierzehnten Tag in Kultur werden die Zellen erneut, aber diesmal mit 6 ml PBS, 3 ml Trypsin und 6 ml Inkubationsmedium, passagiert. Die Zellsuspension wird 5 Minuten zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Die Zellen werden weiter kultiviert und so oft passagiert und ausgesät, bis die Zellen einen semikonfluenten bis konfluenten Zustand erreichen.

### REFERENZBEISPIEL 2

Pankreas-Acini wurden von männlichen Sprague-Dawley-Ratten (20-300 g) erhalten, die narkotisiert (CO₂) und über die dorsale Aorta ausgeblutet worden waren. Eine Kanüle wurde trasduodenal in den Pankreasgang eingeführt und dem Pankreas wurde von hinten 10 ml Digestionsmedium, enthaltend HEPES-Eagle-Medium (pH 7,4), 0,1 mM HEPES-Puffer (pH, 7,6), 70% (Vol./Vol.) modifiziertes Eagle-Medium, 0,5 % (Vol./Vol.) Trasylol (Bayer AG, Leverkusen, Deutschland), 1 % (Gew./Vol.) Rinderserumalbumin), 2,4 mM CaCl₂ und Kollagenase (0,63 P/mg, Serva, Heidelberg, Deutschland) injiziert.

Vor der Entfernung wurde der Pankreas von anhaftendem Festgewebe, Lymphknoten und Blutgefäßen teilweise befreit. Dann wurde gesundes Pankreasgewebe in Digestionsmedium abgeholt (bei 20 °C, geringerer Stoffwechsel), das Pankreasgewebe mit einer Schere sehr fein zerkleinert, oben schwimmendes Fettgewebe abgesaugt und die Gewebesuspension mit Carbogen (Messer, Krefeld, Deutschland) begast, ohne dass die Düse in das Medium mit den Zellen gelangt (Verringerung von mechanischem Streß) und damit auf pH 7,4 eingestellt. Danach wurde die Suspension in einem 25-ml-Erlenmeyerkolben (mit Alufolie bedeckt) unter konstantem Schütteln (150-200 Zyklen pro Minute) bei 37 °C in 10 ml Digestionsmedium inkubiert. Nach 15-20 Minuten wurde das oben schwimmende Fett und das Medium abgesaugt und das Gewebe wurde erneut zerkleinert und mit Medium ohne Kollagenase gespült (Vorgang mindestens zweimal wiederholen, vorzugsweise solange bis Zellfraktion transparent), worauf Digestionsmedium zugegeben und erneut etwa 1 Minute lang mit Carbogen begast wurde. Es folgte wiederum eine Digestion mit Kollagenase für 15 Minuten bei 37°C im Schüttler unter Verwendung desselben Puffers. Nach der Digestion wurden die Acini durch sukzessives Hochziehen und Ausstossen durch 10 ml-, 5 ml- und 2 ml-Glaspipetten mit engen Öffnungen dissoziiert und durch ein einlagiges Nylonsieb (Polymon PES-200/45, Angst & Pfister AG, Zürich, Schweiz) mit einer Maschengröße von etwa 250 µm filtriert. Die Acini wurden zentrifugiert (bei 37°C und 600-800 UpM in einer Beckman-GPR-Zentrifuge, entspricht etwa 50-100 g) und weiter gereinigt durch Waschen in Inkubationsmedium, enthaltend 24,5 mM HEPES (pH 7,5), 96 mM NaCl, 6 mM KCl, 1 mM MgCl₂, 2,5 mM NaH₂PO₄, 0, mM CaCl₂, 11,5 mM Glucose, 5 mM Natriumpyruvat, 5 mM Natriumglutamat, 5 mM Natriumfumarat, 1 % (Vol./Vol.) modifiziertes Eagle-Medium, 1 % (Gew./Vol.) Rinderserumalbumin, mit Carbogen äquilibriert und auf pH 7,4 eingestellt. Die Waschprozedur (Zentrifugation, Absaugen, Resuspension) wurde fünfmal wiederholt. Soweit nicht anders angegeben, wird bei der obigen Isolierung bei etwa 20 °C gearbeitet.

Die Acini wurden in Inkubationsmedium resuspendiert und bei 37°C in einer angefeuchten Atmosphäre mit 5 % CO₂ kultiviert. Das acinäre Gewebe starb dabei schnell (innerhalb von zwei Tagen) ab und die sterbenden differenzierten Zellen lösten sich dabei von den benachbarten Zellen, ohne diese zu schädigen (schonende Isolierung), die nicht absterbenden Stammzellen sanken zu Boden und hefteten sich an. Die differenzierten Acinizellen sind dazu nicht in der Lage. Das Inkubationsmedium wurde am zweiten oder dritten Tag nach dem Aussäen erstmals gewechselt, wobei ein Großteil der frei schwimmenden Acini und acinären Zellen entfernt wurde. Zu diesem Zeitpunkt hatten sich die ersten Stammzellen bzw. deren Vorläufer am Boden festgesetzt und begannen sich zu teilen. Der Mediumwechsel wurde danach an jedem dritten Tag wiederholt und differenzierte acinäre Pankreaszellen wurden bei jedem Mediumwechsel entfernt.

Am siebenten Tag in Kultur wurden die Zellen mit einer Lösung bestehend aus 2 ml PBS, 1 ml Trypsin (+ 0,05 % EDTA) und 2 ml Inkubationsmedium passagiert. Dabei lösen sich die Zellen vom Boden der Kulturschale. Die Zellsuspension wurde 5 Minuten bei etwa 1000 UpM (Beckmann GPR-Zentrifuge) zentrifugiert, der Überstand abgesaugt und die Zellen in 2 ml Inkubationsmedium resuspendiert, auf eine mittlere Zellkulturflasche überführt und 10 ml Inkubationsmedium dazugegeben.

Am vierzehnten Tag in Kultur wurden die Zellen erneut, aber diesmal mit 6 ml PBS, 3 ml Trypsin/EDTA und 6 ml Inkubationsmedium, passagiert. Die Zellsuspension wird 5 Minuten bei 1000 UpM zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Am Tag 17 erfolgte ein drittes Passagieren auf insgesamt 6 mittlere Zellkulturflaschen und am Tag 24 ein viertes Passagieren auf insgesamt 12 mittlere Zellkulturflaschen. Spätestens jetzt waren alle primären Zellen bis auf die Stammzellen aus der Zellkultur entfernt.

Die Stammzellen können weiter kultiviert werden und so oft passagiert und ausgesät wie gewünscht. Das Aussäen erfolgt vorzugsweise jeweils in einer Dichte von 2-4 x 10⁵ Zellen/cm² in Inkubationsmedium.

### BEISPIEL 1

Die Isolierung und Kultivierung aus exokrinem Gewebe der Ohrspeicheldrüse erfolgte analog dem Pankreas-Protokoll mit den folgenden Abweichungen:
1. Das exokrine Gewebe der Ohrspeicheldrüse war eine Mischung von acinärem Gewebe und tubulösem Gewebe.
2. Nachdem Speicheldrüsen weniger Proteasen und Amylasen als Pankreas enthalten, ist es möglich, das Speicheldrüsengewebe vor der Aufarbeitung einige Zeit im Kühlschrank bei etwa 4°C aufzubewahren, ohne dass das Gewebe zu sehr geschädigt wird. Im konkreten Beispielsfall betrug die Aufbewahrungszeit 15 h und brachte keine nachteiligen Folgen für die Isolierung der gewünschten Stammzellen mit sich.

Die folgenden Beispiele 2 und 3 beschreiben detailliert zwei Arbeitsprotokolle zur Herstellung von organoiden Körpern (Organoid Bodies) und differenzierten Zellen.

### BEISPIEL 2

Die undifferenzierten Zellen werden mit einer Lösung aus 10 ml PBS, 4 ml Trypsin, 8 ml Differenzierungsmedium abtrypsiniert und 5 Minuten abzentrifugiert. Das resultierende Pellet wird so in Differenzierungsmedium resuspendiert, dass sich eine Verdünnung von 3000 Zellen je 100 µl Medium einstellt. Anschließend werden die Zellen nochmals gut mit einer 3 ml Pipette suspendiert.

Von bakteriologischen Petrischalen, die vorher mit jeweils 15 ml PBS (37 °C) pro Platte beschichtet worden sind, wird der Deckel abgenommen und umgedreht. Mit Hilfe einer automatischen Pipette werden auf einen Deckel ca. fünfzig 20 µl Tropfen gegeben. Der Deckel wird dann schnell umgedreht und auf die mit Differenzierungsmedium gefüllte Petrischale gegeben, sodass die Tropfen nach unten hängen. Die Petrischalen werden anschließend vorsichtig in den Brutschrank gestellt und für 48 h inkubiert.

Daraufhin werden die in den hängenden Tropfen aggregierten Zellen, die hier Organoid Bodies (OB) genannt werden sollen, aus jeweils vier Deckeln in je eine bakteriologische Petrischale mit 5 ml Inkubationsmedium mit 20 % FKS überführt und für weitere 96 h kultiviert.

Die Organoid Bodies werden nun vorsichtig mit einer Pipette aufgesammelt, und in mit 0,1 % Gelatine beschichtete Zellkulturgefäße mit Differenzierungsmedium überführt. In einer besonders bevorzugten Ausgestaltung des Verfahrens verwendet man als Kulturgefäß mit 0,1 % Gelatine beschichtete 6 cm Petrischalen, in die 4 ml Differenzierungsmedium vorgelegt wurde und die anschließend mit je 6 Organoid Bodies beschickt werden. Ein weiteres bevorzugtes Kulturgefäß sind mit 0,1 % Gelatine beschichtete Chamber Slides, in die 3 ml Differenzierungsmedium vorgelegt wurde und die anschließend mit je 3-8 Organoid Bodies beschickt werden. Daneben können auch 24-Mulden-Mikrotiterplatten verwendet werden, die mit 0,1 % Gelatine beschichtet wurden und in die je 1,5 ml pro Mulde Differenzierungsmedium vorgelegt wurde und anschließend mit je 4 Organoid Bodies beschickt werden.

Derart kultiviert, ist die Differenzierungsfähigkeit der Zellen in den Organoid Bodies aktiviert und die Zellen differenzieren sich in Zellen der drei Keimblätter Mesoderm, Entoderm und Ektoderm. Die Zellen können sowohl als Organoid Bodies als auch als einzelne Zellen gelagert und kultiviert werden und behalten ihre Pluripotenz.

### BEISPIEL 3

Für die Induktion der Differenzierung wurden vorzugsweise Stammzellen nach dem 42. Tag der Kultivierung verwendet. Die Verwendung von Stammzellen nach der 3. oder 4. Passage oder von Zellen, die bei der Temperatur von flüssigem Stickstoff 12-18 Monate lang gelagert worden waren, war ebenfalls problemlos möglich.

Zunächst wurden die Zellen in Differenzierungsmedium mit der oben angegebenen Zusammensetzung überführt und auf eine Dichte von etwa 3 x 10⁴ Zellen/ml eingestellt; z.B. durch Trypsinbehandlung einer Stammzellkultur in Nährmedium, 5-minütige Zentrifugation bei 1000 UpM und Resuspendierung des Pellets in Differenzierungsmedium und Verdünnung soweit erforderlich.

Anschließend wurden mit einer 20-µl-Pipette ca. 50 20-µl-Tropfen (600 Zellen/20 µl) auf die Innenseite des Deckels einer bakteriologischen Petrischale gegeben (gestopfte Spitzen) und die Deckel vorsichtig auf die mit PBS gefüllten Petrischalen gestülpt, sodass die Tropfen nach unten hängen. Für jeden Deckel wurde eine neue Spitze verwendet. Die Petrischalen wurden anschließend vorsichtig in den Brutschrank gestellt und 48 h lang bei 37°C inkubiert.

Danach wurden die in den hängenden Tropfen aggregierten Zellen, die Organoid Bodies (OB), aus jeweils vier Deckeln in je eine bakteriologische Petrischale mit 5 ml Inkubationsmedium mit 20 % FKS überführt (Deckel schräg halten und die OBs mit etwa 2,5 ml Nährmedium abspülen) und für weitere 5-9 Tage, vorzugsweise 96 h, kultiviert.

Die Organoid Bodies wurden nun vorsichtig mit einer Pipette aufgesammelt und in mit 0,1 % Gelatine beschichtete Zellkulturgefäße mit Differenzierungsmedium überführt. Die OB vermehrten sich nun und wuchsen in zum Teil einzelnen Zelkolonien, die wieder vermehrt, vereinzelt und vermehrt werden konnten. In einer besonders bevorzugten Ausgestaltung des Verfahrens wurden als Kulturgefäß mit 0,1 % Gelatine beschichtete 6 cm Petrischalen verwendet, in die 4 ml Differenzierungsmedium vorgelegt worden war, und diese mit je 6 Organoid Bodies beschickt. Ein weiteres bevorzugtes Kulturgefäß waren mit 0,1 % Gelatine beschichtete Chamber Slides, in die 3 ml Differenzierungsmedium vorgelegt wurde und die anschließend mit je 3-8 Organoid Bodies beschickt wurden, und Thermanox-Platten (Nalge Nonc International, USA) für elektronenmikroskopische Studien. Ein weitere Alternative waren 24-Mulden-Mikrotiterplatten, die mit 0,1 % Gelatine beschichtet wurden und in die je 1,5 ml pro Mulde Differenzierungsmedium vorgelegt wurde und die anschließend mit je 4 Organoid Bodies beschickt wurden.

In einer bevorzugten Ausgestaltung des Verfahrens wurden OBs etwa 7 Wochen lang in den gelatine-beschichteten 6-cm-Petrischalen kultiviert und danach wurden einzelne OBs mit dem Microdissector (Eppendorf, Hamburg, Deutschland) nach den Anweisungen des Herstellers ausgeschnitten und dann z.B. auf frische 6-cm-Petrischalen, Chamber Slides oder Thermanox-Platten überführt.

### BEISPIEL 4

Zur Isolierung und Kultivierung von humanen adulten Stammzellen wurde humanes Gewebe von erwachsenen Patienten unmittelbar nach einem chirurgischen Eingriff erhalten und sofort aufgearbeitet. Aus dem chirurgisch entfernten Gewebe, z.B. Speicheldrüsengewebe, wurde gesundes Gewebe abgetrennt und in Digestionsmedium aufgenommen. Dieses Gewebe wurde dann analog dem für die Ratte beschriebenen Protokoll aufgearbeitet und die Stammzellen in analoger Weise isoliert und kultiviert.

Falls die Stammzellen später für therapeutische Zwecke genutzt werden sollen, sind aus Sicherheitsgründen noch verschiedene Bedingungen zu erfüllen, um eine Gefährdung des zu behandelnden Patienten auszuschließen, insbesondere:
- Verwendung von humanem Serum, vorzugsweise Eigenplasma des Patienten, an Stelle von FKS, erforderlichenfalls Aufreinigung des Serums bzw. Plasmas
- Ausschluss jeder tierischen Quelle von weiteren Medienzusätzen
- höchste Reinheit aller Substanzen, Sterilität von Geräten und Umgebung
- Sterilität und Reinheit der Stammzellkultur durch mehrmaliges Passagieren der Stammzellen und Kontrolle auf Kontamination durch Mycoplasmen oder andere Mikrorganismen
- sorgfältige Überprüfung des Ausgangsgewebes und der Stammzellen auf Tumorgenizität.

### BEISPIEL 5

### Charakterisierung differenzierter Zellen

### 1. Immunohistochemie

Organoid Bodies, die mindestens 3 Wochen lang auf Chamber Slides kultiviert worden waren, sowie Querschnitte von "Langzeit"-OBs wurden zweimal in PBS gespült, fünf Minuten lang mit Methanol:Aceton (7:3), enthaltend 1 g/ml DAPI (Roche, Schweiz) bei -20°C fixiert und dreimal in PBS gewaschen. Nach Inkubation in 10%igem normalem Ziegenserum bei Raumtemperatur für 15 Minuten wurden die Proben über Nacht mit primären Antikörpern bei 4°C in einer Befeuchtungskammer inkubiert. Die primären Antikörper waren gegen das Proteingenprodukt 9.5 (PGP 9.5, polyklonaler Kaninchenantikörper, 1:400, Ultraclone, Insel Wight), Neurofilamente (NF-Pan-Cocktail, polyklonaler Kaninchenantikörper, 1:200, Biotrend, Deutschland), α-Smooth-muscle-actin (α-SMA, monoklonaler Mausantikörper, 1:100, DAKO, Dänemark), gliales fibrilläres saures Protein (GFAP, monoklonaler Mausantikörper, 1:100, DAKO, Dänemark), Kollagen II (monoklonaler Mausantikörper II-II-6B3, 1:20, Developmental Studies Hybridoma Bank, University of Iowa, USA), Vigilin FP3 (1:200, Kügler et al., 1996), Cytokeratine (Pan Cytokeratin, monoklonaler Mausantikörper, 1:100, Sigma, USA), Alpha-Amylase (polyklonaler Kaninchenantikörper, 1:100, Calbiochem, Deutschland) und Insulin (monoklonaler Mausantikörper, 0,5 g/ml, Dianova, Deutschland) gerichtet. Nach dreimaligem Spülen mit PBS wurden Objektträger 45 Minuten lang bei 37°C mit entweder Cy3-markiertem Antimaus-IgG oder FITC-markiertem Antikaninchen-IgG (Dianova), beide 1:200 verdünnt, inkubiert. Die Objektträger wurden dreimal in PBS gewaschen, mit Vectashield Mounting Medium (Vector, USA) beschichtet und mit einem Fluoreszenzmikroskop (Axiosop Zeiss, Deutschland) oder mit einem konfokalen Laserscanningmikroskop (LSM 510 Zeiss, Deutschland) analysiert. Eine Alcianblaufärbung wurde mittels Standardverfahren durchgeführt.

### 2. Transmissionselektronenmikroskopie

OBs wurden auf Thermanox-Platten (Nalge Nonc International, USA) mindestens 3 Wochen lang kultiviert. An den Thermanox-Platten haftende Proben wurden durch Eintauchen in 0,1 M Cacodylat-Puffer, enthaltend 2,5 % Glutaraladehyd und 2 % Para-formaldehyd, bei pH 7,4 24 h lang inkubiert. Nach einer Nachfixierung in 1% OsO₄, "en bloc"-Anfärbung mit 2% Uranylacetat und Dehydrierung in reinen Alkoholen wurden die Proben in Araldite eingebettet. Nach Entfernung der Thermanox-Platte wurden Semithin-Schnitte entweder tangential oder senkrecht zur eingebetteten Zellkultur vorgenommen und mit Methylenblau und Azur II geschnitten. Ultradünne Schnitte wurden aus den Regionen von Interesse ausgeschnitten, mit Bleicitrat angefärbt und unter einem Transmissionselektronenmikroskop (Phillips, EM 109) untersucht.

## Patentansprüche

1. Verfahren zur Herstellung adulter pluripotenter Stammzellen aus exokrinem Drüsengewebe, bei dem es sich um exokrines Drüsengewebe aus einer Speicheldrüse, Tränendrüse, Schweißdrüse oder Talgdrüse handelt, welche pluripotenten Stammzellen in der Lage sind, nach Kultivierung unter räumlichen Bedingungen, welche für einen dreidimensionalen Kontakt der Zellen sorgen, in einem Kultivierungsmedium, das keine zusätzlichen Wachstums- oder Differenzierungsfaktoren enthält, in Zelltypen aller drei Keimblätter zu differenzieren,
**dadurch gekennzeichnet, dass** exokrines Drüsengewebe mechanisch und enzymatisch so zerkleinert wird, dass die Zellverbände in den resultierenden Gewebestückchen weitgehend erhalten bleiben,
das zerkleinerte Gewebe in adhärenter Kultur kultiviert und
die in Kultur persistierenden adhärenten Zellen weiter kultiviert und nicht-adhärente differenzierte Zellen durch Mediumwechsel abgetrennt werden,
und adulte pluripotente Stammzellen aus der Kultur gewonnen werden.

2. Verfahren nach Anspruch 1, **dadurch gekennzeichnet, dass**
das zerkleinerte Gewebe zunächst unter geeigneten Bedingungen in Gewebekulturgefäßen kultiviert wird, wobei der Großteil der differenzierten Zellen schnell im Verlauf weniger Tage abstirbt und sich von den Stammzellen löst, wonach die Stammzellen sich am Boden der Gewebekulturgefäßes festsetzen,
und das restliche Gewebe und nicht-adhärente differenzierte Zellen durch einen ersten Mediumwechsel weitgehend abgetrennt werden, und
durch weitere Mediumwechsel in Abständen von wenigen Tagen, vorzugsweise etwa 2-3 Tagen, restliche nicht-adhärente Zellen abgetrennt werden.

3. Verfahren nach einem der Ansprüche 1-2, **dadurch gekennzeichnet, dass** das exokrine Drüsengewebe von einem Wirbeltier, vorzugsweise einem Säuger, stammt.

4. Verfahren nach Anspruch 3, **dadurch gekennzeichnet, dass** das exokrine Drüsengewebe von einem Primaten, insbesondere einem Menschen, stammt.

5. Verfahren nach einem der Ansprüche 1-4, **dadurch gekennzeichnet, dass** das exokrine Drüsengewebe acinäres Gewebe ist.

6. Verfahren nach Anspruch 5, **dadurch gekennzeichnet, dass** das acinäre Gewebe aus der Ohrspeicheldrüse oder Unterkieferspeicheldrüse stammt.

7. Verfahren nach einem der Ansprüche 1-6, **dadurch gekennzeichnet, dass** die Stammzellen das Vermögen zur Bildung von dreidimensionalen Zellverbänden, sogenannten organoiden Körpern, aufweisen.

8. Verfahren nach einem der Ansprüche 1-7, **dadurch gekennzeichnet, dass** die Stammzellen auch nach dem Einfrieren/Kryokonservieren ihre Fähigkeit zur Selbsterneuerung und uneingeschränkten Teilung behalten und nicht differenzieren.

## Claims

1. A method of producing adult pluripotent stem cells from exocrine glandular tissue, which exocrine glandular tissue is derived from a salivary gland, lacrimal gland, sudoriferous gland or sebaceous gland, and which pluripotent adult stem cells are - after culturing under spatial conditions which ensure three-dimensional contact of the cells - capable of differentiating into cell types of all three germ layers in a culture medium that does not contain any additional growth factors or differentiation factors, **characterized in that** exocrine glandular tissue is mechanically and enzymatically divided in such a manner that the cell structures in the resulting tissue fragments are largely preserved,
the divided tissue is cultured in an adherent culture, and the adherent cells persisting in the culture are further cultured and nonadherent differentiated cells are separated by a change of medium,
and adult pluripotent stem cells are obtained from the culture.

2. The method according to Claim 1, **characterized in that** the divided tissue is first cultured under suitable conditions in tissue culture vessels, whereby most of the differentiated cells rapidly die in the course of a few days and become detached from the stem cells, whereupon the stem cells adhere on the bottom of the tissue culture vessel,
and the remaining tissue and nonadherent differentiated cells are largely separated by a first change of medium and the remaining nonadherent cells are separated by additional changes of medium at intervals of a few days, preferably about 2 to 3 days.

3. The method according to one of claims 1-2, **characterized in that** the exocrine glandular tissue originates from a vertebrate, preferably a mammal.

4. The method according to claim 3, **characterized in that** the exocrine glandular tissue originates from a primate, in particular a human.

5. The method according to one of claims 1-4, **characterized in that** the exocrine glandular tissue is acinar tissue.

6. The method according to claim 5, **characterized in that** the acinar tissue is derived from the parotid gland or the mandibular gland.

7. The method according to one of claims 1-6, **characterized in that** the stem cells are capable to form three-dimensional cell structures, so-called organoid bodies.

8. The method according to one of claims 1-7, **characterized in that** even after freezing/cryopreservation the stem cells still retain their ability for self-renewal and unlimited division and do not differentiate.

## Revendications

1. Procédé de production de cellules souches pluripotentes adultes à partir de tissu glandulaire exocrine dans lequel il s'agit de tissu glandulaire exocrine choisi parmi une glande salivaire, une glande lacrymale, une glande sudoripare ou une glande sébacée, lesdites cellules souches pluripotentes étant capables de se différencier, après la culture dans des conditions ambiantes qui assurent un contact tridimensionnel des cellules, dans un milieu de culture qui ne contient pas de facteurs de croissance ou de différenciation supplémentaires en type de cellules de l'ensemble des trois feuillets embryonnaires,
**caractérisé en ce que** le tissu glandulaire exocrine est réduit de façon mécanique et enzymatique de telle sorte que les structures cellulaires restent largement conservées dans les petits morceaux de tissus obtenus,
le tissu réduit est cultivé en culture adhérente et
les cellules adhérentes persistant dans la culture sont encore cultivées et les cellules différenciées non adhérentes sont séparées par un échange de milieu,
et des cellules souches pluripotentes adultes sont obtenues à partir de la culture.

2. Procédé selon la revendication 1, **caractérisé en ce que** le tissu réduit est tout d'abord cultivé dans des conditions appropriées dans des récipients de culture tissulaire, la majeure partie des cellules différenciées mourant rapidement en l'espace de quelques jours et se détachant des cellules souches, après quoi les cellules souches se fixent sur le fond du récipient de culture cellulaire,
et le tissu restant et les cellules différenciées non adhérentes sont largement séparées par un premier changement de milieu, et
les cellules non adhérentes restantes sont séparées par un autre changement de milieu à intervalles de quelques jours, de préférence, environ 2 à 3 jours.

3. Procédé selon l'une des revendications 1 à 2, **caractérisé en ce que** le tissu glandulaire exocrine provient d'un animal vertébré, de préférence, d'un mammifère.

4. Procédé selon la revendication 3, **caractérisé en ce que** le tissu glandulaire exocrine provient d'un primate, en particulier d'un être humain.

5. Procédé selon l'une des revendications 1 à 4, **caractérisé en ce que** le tissu glandulaire exocrine est un tissu acinaire.

6. Procédé selon la revendication 5, **caractérisé en ce que** le tissu acinaire provient de la glande parotide ou de la glande submandibulaire.

7. Procédé selon l'une des revendications 1 à 6, **caractérisé en ce que** les cellules souches présentent la capacité de former des structures cellulaires tridimensionnelles, nommées corps organoïdes.

8. Procédé selon l'une des revendications 1 à 7, **caractérisé en ce que** les cellules souches conservent également après leur congélation/ cryoconservation, leur capacité à s'auto-renouveler et à se diviser de façon illimitée et non de se différencier.
